# EUROPEAN PATENT SPECIFICATION

(11) **EP 2 688 888 B1**
(45) Date of publication and mention of the grant of the patent: **14.06.2017**
(21) Application number: 11862192.9
(22) Date of filing: 25.03.2011
(51) Int. Cl.: C07D 487/04, A61K 31/519, A61P 35/00

(54) **PROCESS FOR THE PRODUCTION OF DISODIUM PEMETREXED**
VERFAHREN ZUR HERSTELLUNG VON DI-NATRIUM PEMETREXED
PROCÉDÉ POUR LA PRODUCTION DE DISODIUM PÉMÉTREXED

(43) Date of publication of application: 29.01.2014
(73) Proprietor: Scinopharm Taiwan, Ltd., Taiwan County 741 (TW)
(72) Inventor: CHEN, ShangHong, Chiayi City (TW); LIN, HuangHsiung, Tainan County (TW)
(74) Representative: Gulde & Partner
(86) International application number: PCT/SG2011/000122
(87) International publication number: WO 2012/134392

(56) References cited:
- EP-A1- 0 905 128
- EP-A1- 2 213 674
- WO-A1-99/16742
- WO-A2-01/14379
- WO-A2-01/62760
- WO-A2-2010/028105

## Description

### BACKGROUND OF THE INVENTION

### 1. Field of the Invention

The present application relates to process of making a pemerexed disodium.

### 2. Description of the Related Art

Pemetrexed and salts thereof are known as anti-folate, anti-neoplastic agents. Pemetrexed's chemical name is (S)-2-[4-[2-(4-amino-2-oxo-3,5, 7-triazabicyclo[4.3.0]nona-3,8, 10-then-9-yl)ethyl]benzoyl]amino-pentanedioic acid and has the following chemical structure:

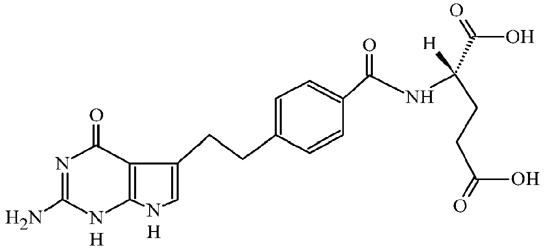

The most common salt of pemetrexed is a disodium salt, i.e., pemetrexed disodium. Pemetrexed disodium has the chemical name L-Glutamic acid, *N*-[4-[2-(2-amino-4,7-dihydro-4-oxo-1*H*-pyrrolo[2,3-*d*]pyrimidin-5-yl)ethyl]benzoyl]-, disodium salt. Pemetrexed disodium heptahydrate is the active ingredient of Eli Lilly and Company's ALIMTA® injectable compsostiion. Pemetrexed disodium heptahydrate has the following chemical structure:

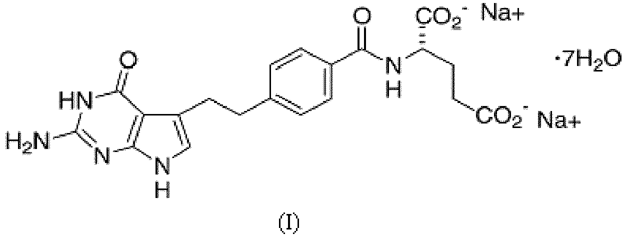

Various methods for preparing pemetrexed and pemetrexed disodium are disclosed in the art, such as International Patent Application Publication Nos. WO2001014379 and WO1999016742.

However, there is still a need for an improved process of making a pemetrexed salt.

### SUMMARY OF THE INVENTION

The present application provides a process of making a pemetrexed disodium comprising:
a) reacting a compound of formula II 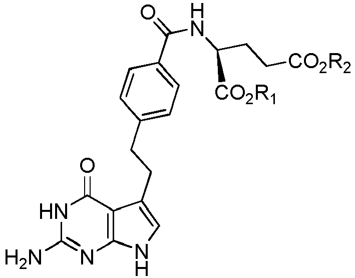 or an acid salt thereof, wherein each of R₁ and R₂ is independently a C₁-C₆ alkyl group, with sodium hydroxide solution at a temperature of no more than 10°C to obtain a first mixture;
b1) adding an organic anti-solvent to the first mixture of step a) to form a second mixture;
b2) adjusting the pH of the second mixture within a range from about 6.5 to about 9.5 to form a third mixture;
b3) separating the pemextrexed disodium from the third mixture.

The steps b1)-b2) may be conducted at no more than 10°C, more preferably no more than 5°C.

Preferably, the step a) is conducted at no more than 5°C.

The organic anti-solvent may be any suitable organic solvent, preferably a water miscible solvent, more preferably a solvent selected from isopropanol, propanol, acetone, methanol, ethanol, acetonitrile, THF, and combinations thereof.

The acid salt of the compound of formula (II) may be any suitable salt with at least one counter anion. For example, the acid salt may be selected from p-TSA (p-toluenesulfonic acid), sulfuric, hydrochloric, hydrobromic, phosphoric, hypophosphoric, hydroiodic, sulfamic, citric, acetic, maleic, malic, succinic, tartaric, cinnamic, benzoic, ascorbic, mandelic, benzensulfonic, methanesulfonic, trifluoroacetic, hippuric salts, and combinations thereof. Preferably, the acid salt is a p-TSA salt.

Each of R₁ and R₂ in formula (II) is preferably an ethyl group.

The crude pemetrexed disodium obtained in accordance with the above-described process may be further recrystallized, preferably at a temperature of 15 to 30°C, more preferably at a temperature of 20-25°C.

In step b), the pH of the resulting solution is preferably adjusted to from about 7.5 to about 8.5. The pH discussed in the step b) is preferably adjusted by a pH adjusting solution, e.g., hydrochloric acid in a suitable solvent, preferably a water miscible solvent, more preferably a solvent selected from isopropanol, propanol, water, acetonitrile, methanol, ethanol, THF, and acetone, more preferably a pH adjusting aqueous solution.

The various features of novelty which characterize the invention are pointed out with particularity in the claims annexed to and forming a part of the disclosure. For a better understanding of the invention, its operating advantages, and specific objects attained by its use, reference should be had to the descriptive matter in which there are illustrated and described preferred embodiments of the invention.

### DETAILED DESCRIPTION OF THE PRESENTLY PREFERRED EMBODIMENTS

The following embodiments are provided to further explain, but not to limit, the present invention.

As discussed above, the pemetrexed disodium is formed from the reaction between compound of formula (II) and the sodium hydroxide solution.

The present application discloses a method for the preparation of crude pemetrexed disodium, which may be further purified to pemetrexed disodium 2.5 H₂O crystalline form. In accordance with an emboidment of the present invention, the aforesaid preparation method may comprise a hydrolysis step of formula (II) to furnish crude pemetrexed disodium at a low temperature followed by the addition of IPA and then the pH is adjusted by adding hydrochloric acid.

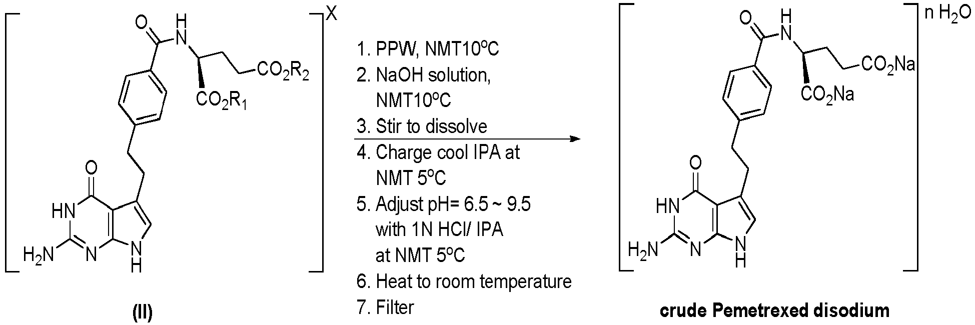

In the above Formula (II), each of R₁ and R₂ is independently a C1-C6 alkyl group, such as ethyl, X is a counter anion so that the compound of formula (II) is an acid salt, such as a p-TSA salt, n represents the number of water, which typically ranges from 2.5 to 7.0.

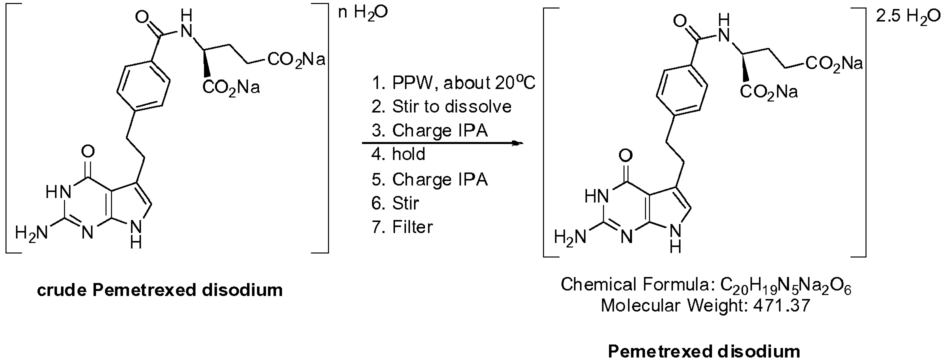

In comparsion with the prior art, the embodiments of the present invention have the following advantages:
1) The reaction of the compound of formula (II) or an acid salt thereof with sodium hydroxide solution is preferably conducted at a low temperature, e.g., no more than 10°C, in comparison with the room temperature of WO2001014379 and WO1999016742. As shown in Table 1 shown below, the inventors of the present invention unexpectedly discovered that impurities can be reduced or minimized by conducting the reaction at a low temperature. As noted above, after the reaction step, the low temperaure is preferably maintained during the steps of adjusting pH and adding anti-sovlent.
2) The anti-solvent is preferably added before adjusting the pH value. The inventors unexpectedly discovered that by doing so, the mixture can be diluted by the addition of the anti-solvent before the adjusting step, therefore minimizing local heat effect and formation of impurities. In comparison, WO2001014379 discloses adjusting the pH value before the addition of an anti-solvent.
3) In accordance with the present invention, the compound of formula (II) can be converted to a pemetrexed disodium (e.g., at a pH of 6.5 to 9.5). In contrast, the process of WO1999016472 comprises making pemetrexed free form first (at a pH of about 3), and then converting the free form to a salt (at a pH of about 6.5 to 9.5). The process of the present invention is simpler and more convenient.
4) In accordance with an embodiment of the present invention, the crude pemetrexed disodium may be crystallized at a room temperature; whereas in accordance with WO2001014379, the crystallizing is conducted at about 70°C. The cooling, heating, and adjusting pH steps of WO2001014379 may be avoided in accordance with the embodiment of the present invention. The process of the present invention can save workup procedures and has better control of impurity.

**Table 1 The stability of pemetrexed disodium in sodium hydroxide at different temperature**

| Rex. Temperature (°C) | 1 hr* (%) | 2 hr* (%) | 4 h* (%) | 6 hr* (%) |
|---|---|---|---|---|
| 5 | 0.012 | 0.013 | 0.035 | 0.066 |
| 10 | 0.051 | 0.053 | 0.070 | 0.075 |
| 15 | 0.076 | 0.085 | 0.110 | 0.123 |

| | | | | |
|---|---|---|---|---|
| * The content of impurity based on HPLC spectra's % Area. | | | | |

### Example 1 Preparation of crude pemetrexed disodium

N-[4-2-(2-Amino-4, 7-dihydro-4-oxo-1H-pyrrolo[2,3-d]pyrimidin-5-yl)ethyl]benzoyl]-L-glutamic Acid Diethyl Ester 4-Methylbenzenesulfonic Acid Salt and purified process water (PPW) (about 10 kg) are charged to a suitable vessel under nitrogen. The reactor is cooled to NMT 10°C under nitrogen. Pre-cooled sodium hydroxide solution (about 1.5 kg)/PPW (about 11.4 kg) are added and the temperature is maintained at NMT 10°C. The mixture is stirred at NMT 10°C until the solid is dissolved. Pre-cooled isopropanol (about 62.8 kg) is added and the mixture temperature is maintained at NMT 5°C. Pre-cooled 1 N hydrochloric acid in isopropanol is added to adjust the pH to 6.5 to 9.5, preferably between pH 7.5 to pH 8.5, at NMT 5°C. The mixture is warmed to a room temperature (i.e., 15-30°C, preferably 20-25°C) and stirred. The solids are filtered and washed with isopropanol/PPW. The wet cake is vacuum dried to provide crude pemetrexed disodium (about 2.30 kg).

### Example 2 Purification of crude pemetrexed disodium to pemetrexed disodium

Crude pemetrexed disodium (about 2.1 kg) and PPW (about 23.3 kg) are charged under nitrogen to a suitable vessel at 15 to 30°C. Isopropanol (about 28.3 kg) is added slowly to cloud point and stirred. Isopropanol (up to about 55 kg) is charged and stirred. The solids are filtered and washed with isopropanol/PPW. The wet cake is vacuum dried to provide pemetrexed disodium (about 1.9 kg) (90% Yield). 1 H NMR (D₂O): δ 7.51 (2H, d, J=8.0 Hz), 6.98 (2H, d, J=8.0 Hz), 6.12 (1 H, s), 4.26-4.23 (H, m), 3.60-3.54 (4H, m), 2.27-2.23 (2H, m), 2.13-2.08 (1 H, m), 2.00-1.94 (1 H, m)

## Claims

1. A process of making a pemetrexed disodium comprising:
a) reacting a compound of formula II 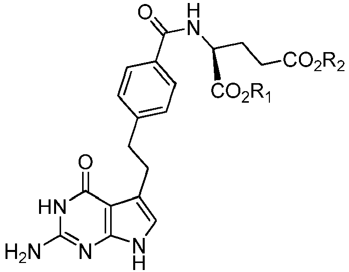 or an acid salt thereof, wherein each of R₁ and R₂ is independently a C1-C6 alkyl group, with sodium hydroxide solution at a temperature of no more than 10°C to obtain a first mixture;
b1) adding an organic anti-solvent to the first mixture of step a) to form a second mixture;
b2) adjusting the pH of the second mixture within a range from about 6.5 to about 9.5 to form a third mixture;
b3) separating the pemetrexed disodium from the third mixture.

2. The process of claim 1 wherein the steps b1)-b2) are conducted at no more than 10°C.

3. The process of claim 1 wherein the steps b1)-b2) are conducted at no more than 5°C.

4. The process of claim 1 wherein the step a) is conducted at no more than 5°C.

5. The process of claim 1 wherein the organic anti-solvent is selected from isopropanol, propanol, acetone, methanol, ethanol, acetonitrile, THF, and combinations thereof.

6. The process of claim 1 wherein the acid salt is selected from p-TSA, sulfuric, hydrochloric, hydrobromic, phosphoric, hypophosphoric, hydroiodic, sulfamic, citric, acetic, maleic, malic, succinic, tartaric, cinnamic, benzoic, ascorbic, mandelic, benzensulfonic, methanesulfonic, trifluoroacetic, hippuric salts or combinations thereof.

7. The process of claim 1 wherein a p-TSA salt of the compound of formula (II) is used in the reacting step.

8. The process of claim 1 wherein each of R₁ and R₂ is an ethyl group.

9. The process of claim 1 further comprising a step of recrystallizing the pemetrexed disodium isolated from the step b3) at a temperature of 15 to 30°C.

10. The process of claim 9 wherein the recrystallizing is conducted in water and an organic anti-solvent.

## Patentansprüche

1. Verfahren zur Herstellung eines Pemetrexeddinatriums, das Folgendes umfasst:
a) Umsetzen einer Verbindung der Formel II 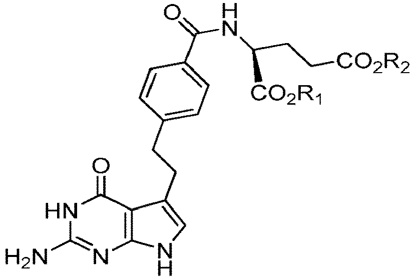 oder eines Säuresalzes davon, wobei R₁ und R₂ jeweils unabhängig voneinander eine C1-C6-Alkylgruppe sind, mit Natriumhydroxidlösung bei einer Temperatur von nicht mehr als 10 °C, um ein erstes Gemisch zu erhalten;
b1) Zugeben eines organischen Anti-Lösungsmittels zu dem ersten Gemisch aus Schritt a), um ein zweites Gemisch zu bilden;
b2) Einstellen des pH des zweiten Gemischs innerhalb eines Bereichs von etwa 6,5 bis etwa 9,5, um ein drittes Gemisch zu bilden;
b3) Abtrennen des Pemetrexeddinatriums von dem dritten Gemisch.

2. Verfahren nach Anspruch 1, wobei die Schritte b1)-b2) bei nicht mehr als 10 °C durchgeführt werden.

3. Verfahren nach Anspruch 1, wobei die Schritte b1)-b2) bei nicht mehr als 5 °C durchgeführt werden.

4. Verfahren nach Anspruch 1, wobei der Schritt a) bei nicht mehr als 5 °C durchgeführt wird.

5. Verfahren nach Anspruch 1, wobei das organische Anti-Lösungsmittel aus Isopropanol, Propanol, Aceton, Methanol, Ethanol, Acetonitril, THF und Kombinationen davon ausgewählt ist.

6. Verfahren nach Anspruch 1, wobei das Säuresalz aus p-TSA-, Schwefel-, Salz-, Bromwasserstoff-, Phosphor-, Unterphosphor-, lodwasserstoff-, Sulfamin-, Zitronen-, Essig-, Malein-, Apfel-, Bernstein-, Wein-, Zimt-, Benzoe-, Ascorbin-, Mandel-, Benzolsulfon-, Methansulfon-, Trifluoressig-, Hippursalzen oder Kombinationen davon ausgewählt ist.

7. Verfahren nach Anspruch 1, wobei ein p-TSA-Salz der Verbindung der Formel (II) in dem Reaktionsschritt verwendet wird.

8. Verfahren nach Anspruch 1, wobei R₁ und R₂ jeweils eine Ethylgruppe sind.

9. Verfahren nach Anspruch 1, das ferner einen Schritt des Rekristallisierens des in Schritt b3) isolierten Pemetrexeddinatriums bei einer Temperatur von 15 bis 30 °C umfasst.

10. Verfahren nach Anspruch 9, wobei das Rekristallisieren in Wasser und einem organischen Anti-Lösungsmittel durchgeführt wird.

## Revendications

1. Procédé de fabrication d'un pemetrexed disodique, comprenant :
a) la mise en réaction d'un composé de formule II 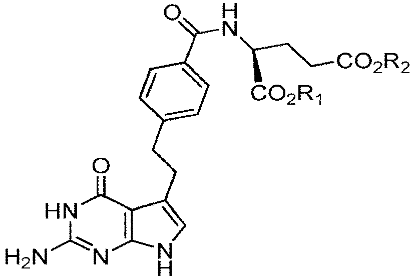 ou d'un sel d'acide de celui-ci, dans lequel chaque radical parmi R₁ et R₂ est indépendamment un groupe alkyl C1-C6, avec une solution d'hydroxyde de sodium à une température ne dépassant pas 10° C pour obtenir un premier mélange ;
b1) l'addition d'un anti-solvant organique au premier mélange de l'étape a) pour former un deuxième mélange ;
b2) l'ajustement du pH du deuxième mélange dans une plage d'environ 6,5 à environ 9,5 pour former un troisième mélange ;
b3) la séparation entre le pemetrexed disodique et le troisième mélange.

2. Procédé selon la revendication 1, dans lequel les étapes b1)-b2) sont réalisées à une température ne dépassant pas 10° C.

3. Procédé selon la revendication 1, dans lequel les étapes b1)-b2) sont réalisées à une température ne dépassant pas 5° C.

4. Procédé selon la revendication 1, dans lequel l'étape a) est réalisée à une température ne dépassant pas 5° C.

5. Procédé selon la revendication 1, dans lequel l'anti-solvant organique est sélectionné parmi l'isopropanol, le propanol, l'acétone, le méthanol, l'éthanol, l'acétonitrile, le THF, et des combinaisons de ceux-ci.

6. Procédé selon la revendication 1, dans lequel le sel d'acide est sélectionné parmi les sels p-TSA, sulfuriques, hydrochloriques, hydrobromiques, phosphoriques, hypophosphoriques, hydroiodiques, sulfamiques, citriques, acétiques, maléiques, maliques, succiniques, tartriques, cinnamiques, benzoiques, ascorbiques, mandéliques, benzènesulfoniques, méthanosulfoniques, trifluoroacétiques, hippuriques ou des combinaisons de ceux-ci.

7. Procédé selon la revendication 1, dans lequel un sel p-TSA du composé de formule (II) est utilisé dans l'étape de réaction.

8. Procédé selon la revendication 1 dans lequel chaque radical parmi R₁ et R₂ est un groupe éthyle.

9. Procédé selon la revendication 1, comprenant également une étape de recristallisation du pemetrexed disodique isolé à partir de l'étape b3) à une température de 15 à 30° C.

10. Procédé selon la revendication 9 dans lequel la recristallisation est réalisée dans de l'eau et dans un anti-solvant organique.
